(19) Europäisches Patentamt
European Patent Office
Office européen des brevets

(11) Publication number: 0 239 641 B1

(12) EUROPEAN PATENT SPECIFICATION

(45) Date of publication of patent specification: 28.03.90

(51) Int. Cl.$^5$: C 07 C 69/675, C 08 F 299/06, C 03 C 25/02, G 02 B 6/44

(21) Application number: 85904668.2

(22) Date of filing: 12.09.85

(86) International application number: PCT/JP85/00509

(87) International publication number: WO 87/01697 26.03.87 Gazette 87/07

# (54) DI(METH)ACRYLIC ESTERS AND RESIN COMPOSITION.

(43) Date of publication of application: 07.10.87 Bulletin 87/41

(45) Publication of the grant of the patent: 28.03.90 Bulletin 90/13

(84) Designated Contracting States: DE FR GB NL

(56) References cited:
JP-A-5 626 844
JP-A-60 193 945
JP-B-5 851 011

No relevant documents have been disclosed.

(73) Proprietor: NIPPON KAYAKU KABUSHIKI KAISHA
11-2, Fujimi 1-chome Chiyoda-ku
Tokyo 102 (JP)

(72) Inventor: YOKOSHIMA, Minoru
2959, Ohazakohri Sanyocho Asa-guun
Yamaguchi 757 (JP)
Inventor: OHKUBO, Tetsuo
92, Ohazanakanokaisaku Ube-shi
Yamaguchi 759-02 (JP)
Inventor: HATTORI, Hideaki
1039, Kamiochiai Yono-shi
Saitama 338 (JP)
Inventor: KIYOMOTO, Masayuki
570, Ohazakohri Sanyocho Asa-gun
Yamaguchi 757 (JP)

(74) Representative: Woods, Geoffrey Corlett
J.A. KEMP & CO. 14 South Square Gray's Inn
London WC1R 5EU (GB)

Note: Within nine months from the publication of the mention of the grant of the European patent, any person may give notice to the European Patent Office of opposition to the European patent granted: Notice of opposition shall be filed in a written reasoned statement. It shall not be deemed to have been filed until the opposition fee has been paid. (Art. 99(1) European patent convention).

EP 0 239 641 B1

Courier Press, Leamington Spa, England.

**Description**

The present invention relates to a diester of (meth)acrylic acid, which can be copolymerized easily with resins containing unsaturated group(s) in the presence of heat, ultraviolet rays, ionizing radiation or a radical-initiator, and resin compositions comprising the same.

The use of optical fibers has increased remarkably in recent years, particularly in the field of communications, because the information-transmitting capacity of optical fibers is large and optical fibers are relatively free from outside interference. Since optical fibers are used in the communications field, they are generally made of glass. However, glass fiber is brittle and is chemically attacked by water vapour; accordingly it is easily broken and is difficult to handle. Therefore, the surface of optical glass fibers has hitherto been coated with a resin.

Resins such as epoxy resin and urethane resin have hitherto been used for such a purpose. However, since they have poor productivity because of their prolonged curing time and since they lack pliability, they have a disadvantage that the transmission specificity of optical glass fibers coated therewith is reduced by side pressure. To overcome this disadvantage, compositions containing urethane acrylate which are curable by ultraviolet rays have been investigated recently; such a composition for optical glass fibers and a method for forming a coating have been proposed, for instance, in JP—A—58—223638 and JP—A—59—170154.

In these applications, a primary coating of a very low modulus has been used to try to overcome the above-mentioned problem, and considerable success has been attained. However, in providing the low modulus, the strength and the hardness of the coating on the glass surface have been sacrificed. Accordingly, it is desirable to form a top coating on the primary coating. Compositions for the top coating which are curable by ultraviolet rays have been investigated. For instance, such a composition is proposed in JP—A—59—170155.

This composition comprises: (1) from 25 to 70 wt% of the coating composition of diethylenic-terminated polyurethane which may include urea linkages, said polyurethane being based on a diisocyanate having an average molecular weight of from 400 to 5000; (2) from 5 to 40 wt% of the coating composition of a diethylenically unsaturated ester of a diglycidyl ether of a bisphenol, said ether having a molecular weight up to about 1000; (3) from 0 to 30 wt% of the coating composition of a diacrylate ester of a polyether glycol, said polyether glycol having a molecular weight less than about 300, and/or a liquid radiation curable monoethylenically unsaturated monomer having a glass transition temperature of 55°C or above; and (4) from 0 to 15 wt% of the coating composition of a triacrylate.

The top coating composition curable by ultraviolet rays which is now in use has a high curing speed, and the desired properties for coating are easily and exactly obtainable. However, such a composition has the disadvantages that the glass fiber is apt to be attacked by water because of the large degree of water-absorption of the coating and the fiber may be broken when rolling up because of the low elongation of the cured coating.

The present invention provides a diester of (meth)acrylic acid represented by the formula (I):

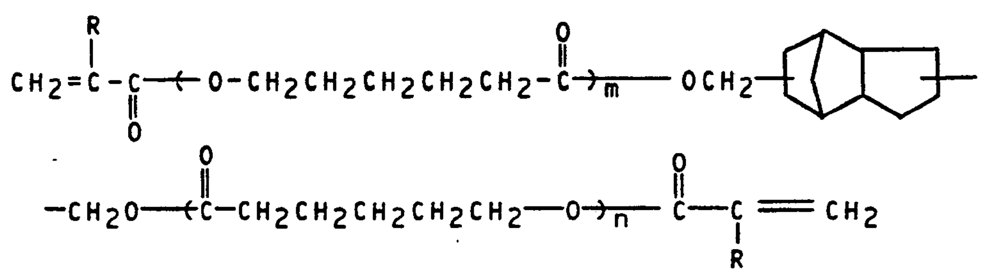

(I)

wherein the mean values of m and n are independently from 0 to 5, preferably from 0 to 3, the mean value of m + n is from 1 to 10, preferably from 1 to 6, and R represents a hydrogen atom or a methyl group.

The present invention also provides a resin composition comprising (A) polyurethane (meth)acrylate(s); (B) diester(s) of (meth)acrylic acid represented by the formula (I); (C) monoethylenically unsaturated monomer(s); and (D) optionally initiator(s) of photopolymerization.

The diester of (meth)acrylic acid can have various uses and the resin composition has a fast curing speed and is suitable for top-coating of optical glass fibers for light transmission. The elongation of the resinous coating obtained by curing the composition is large and the degree of water-absorption of the resinous coating is small.

The diester represented by the formula (I) may be prepared by esterifying a compound represented by the formula (II):

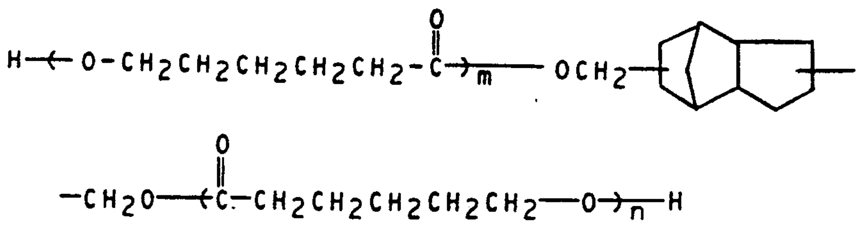

(II)

wherein m and n are as defined above, with acrylic acid or methacrylic acid.

The compound represented by the formula (II), namely, an adduct of tricyclodecanedimethylol and ε-caprolactone, is produced by the reaction between tricyclodecanedimethylol and ε-caprolactone as follows:

During the reaction between tricyclodecanedimethylol and ε-caprolactone, it is preferable to use an effective amount of a catalyst, namely from 0.001 to 1.0 %, preferably from 0.01 to 0.2%, by weight of the ε-caprolactone. Examples of catalyst are organic titanium compounds such as tetraisopropyl titanate and tetrabutyl titanate and tin compounds such as tetraphenyltin, tetraoctyltin, dilauryltin oxide and di-n-butyltin dichloride.

The reaction between tricyclodecanedimethylol and ε-caprolactone is carried out at a temperature of from 50 to 300°C, preferably from 110 to 200°C, for a period sufficient for the completion of the reaction. The amount of ε-caprolactone used in the reaction is from 1 to 10 mol, preferably from 1 to 6 mol, per one mol of tricyclodecanedimethylol. In order to minimize an oxidative side reaction, it is preferable to carry out the reaction in the inert atmosphere such as gaseous nitrogen. The reaction product comprising the adduct of tricyclodecanedimethylol and ε-caprolactone can be utilized directly in the following esterification.

The diester of (meth)acrylic acid represented by the formula (I) is produced by reacting the compound represented by the formula (II) with acrylic acid, methacrylic acid or a mixture thereof. The amount of acrylic acid or methacrylic acid used in the reaction is from 2 to 4 mols per one mol of the compound represented by the formula (II).

Although it is desirable to use the stoichiometrical amount, namely 2 mols of acrylic acid, methacrylic acid or a mixture thereof, to react with the active hydrogen atoms in the terminal hydroxyl groups of one mole of the adduct represented by the formula (II), it is preferable to use a little more than the stoichiometrical amount in order to carry out the reaction completely. It is preferable to carry out the reaction in the presence of a polymerization-inhibitor in order to minimize or retard the polymerization of acrylic compound(s). Such a polymerization-inhibitor is well known by persons skilled in the art, and is used in a concentration of from 0.01 to 5% by weight of the reaction mixture. Examples of the polymerization-inhibitor are hydroquinone, p-methoxyphenol, 2,4-dimethyl-6-t-butylphenol, p-benzoquinone, phenothiazine, N-nitrosodiphenylamine and copper salts. The esterification reaction is generally carried out at a temperature of 50 to 130°C, preferably 65 to 90°C, for a time sufficient to complete the esterification. The period of reaction depends on the scale of the reaction batch, the reactants, the catalyst and the reaction conditions. In addition, an esterification catalyst may be used in a concentration of from 0.1 to 15 mol%, preferably 1 to 6 mol%, of the acrylic acid or methacrylic acid. Any known esterification catalyst may be used, for example, p-toluenesulfonic acid, methanesulfonic acid, phosphoric acid and sulfuric acid. It is preferable to use an inert solvent such as hexane, cyclohexane, benzene and toluene to effect removal of water formed during the esterification.

The diester of (meth)acrylic acid has low irritation, relatively low viscosity and is quickly cured. It can be applied in various uses after mixing with other resins containing unsaturated group(s).

The composition containing the diester(s) of (meth)acrylic acid and resin(s) containing unsaturated group(s) can be cured by heat, ultraviolet rays, ionizing radiation or radical initiators, preferably by ultraviolet rays. In the case where the curing is carried out by ultraviolet rays, an initiator of photopolymerization is added to the composition, preferably at a concentration of from 0.1 to 10% by weight of the composition. Initiators of photopolymerization are well known; examples are benzyl ketal, benzoin isopropyl ether, benzophenone, thioxanthone and anthraquinone. Examples of the resin containing unsaturated group(s), which is usable with the diester of (meth)acrylic acid, are epoxy-acrylates such as acrylic acid ester of epoxidized bisphenol A, acrylic acid ester of epoxidized linseed oil and acrylic acid ester of epoxidized phenol novolak, unsaturated polyesters containing saturated or unsaturated carboxylic acid such as maleic acid, fumaric acid and adipic acid, and urethane acrylates obtained by reacting diisocyanates or polyisocyanates with hydroxyalkyl acrylates.

The composition containing the diester(s) of (meth)acrylic acid can be coated on the surface of any material such as wood, metal, glass, fabric, paper, fiber and plastics having a form of, for instance, a sheet, coil, molded article, film, panel or tube.

The average molecular weight of the polyurethane (meth)acrylate (A) contained in the resin composition according to the present invention is ordinarily not less than 500, and preferably from 500 to 5,000. As such a polyurethane (meth)acrylate, polyurethane (meth)acrylate of a polyether polyol having ether linkage(s) in the molecule thereof, polyurethane (meth)acrylate of a carbonate polyol having

carbonate linkage(s) in the molecule thereof, polyurethane (meth)acrylate of a polyester polyol having ester linkage(s) in the molecule thereof or polyurethane (meth)acrylate having both ether linkage(s) and ester linkage(s) may be mentioned. As the polyether polyol, for instance, polypropylene glycol, polyethylene glycol, polytetramethylene glycol and the compound formed by the addition of ethylene oxide or propylene oxide to, for example, 1,3-butylene glycol, 1,4-butylene glycol, 1,6-hexanediol, neopentyl glycol, cyclohexane dimethanol, 2,2-bis(4-hydroxycyclohexyl)propane, bisphenol A may be used. The polyester polyol can be obtained by reacting an alcoholic compound with an acidic compound. For instance, polypropylene glycol, polyethylene glycol, polytetramethylene glycol or a compound formed by the addition of ethylene oxide, propylene oxide or ε-caprolactone to, for example, 1,3-butylene glycol, 1,4-butylene glycol, 1,6-hexanediol, neopentyl glycol, cyclohexanedimethanol, 2,2-bis(4-hydroxycyclohexyl)propane or bisphenol A may be used as the alcoholic compound. As the acidic compound, a dibasic acid such as phthalic acid, tetrahydrophthalic acid, adipic acid, sebacic acid, azelaic acid, hexahydrophthalic acid, dodecanedicarboxylic acid and an anhydride thereof may be used. In addition, the compound obtained by reacting the alcoholic compound, acidic compound and ε-caprolactone simultaneously may be used as the polyester polyol.

The carbonate polyol can be produced, for instance, by an ester-exchange reaction of a carbonate derivative, for instance, a diaryl- or dialkyl carbonate such as diphenyl carbonate, bis(chlorophenyl) carbonate, dinaphthyl carbonate, phenyl toluyl carbonate, phenyl chlorophenyl carbonate, 2-tolyl 4-tolyl carbonate, dimethyl carbonate or diethyl carbonate with a diol, for instance, 1,6-hexane diol, neopentyl glycol, 1,4-butanediol, 1,8-octanediol, 1,4-bis(hydroxymethyl)cyclohexane, 2-methylpropanediol, dipropylene glycol, dibutylene glycol or a polyester diol formed by reacting one of the diols with a dicarboxylic acid such as oxalic acid, malonic acid, succinic acid, adipic acid, azelaic acid or hexahydrophthalic acid or ε-caprolactone. In addition, the carbonate polyol may be prepared by the reaction of phosgene with one of the diols.

In order to obtain the polyurethane (meth)acrylate (A) while using the above polyether polyol, polycarbonate polyol or polyester polyol, an organic diisocyanate and a polymerizable monomer having hydroxyl group(s) are reacted with the hydroxyl group(s) of the polyol to the extent that the reaction product substantially does not contain any isocyanate group (—NCO). Examples of the organic diisocyanate are aromatic diisocyanates such as tolylene diisocyanate and 4,4'-diphenylmethane diisocyanate, alicyclic diisocyanates such as isophorone diisocyanate and 4,4'-dicyclohexylmethane diisocyanate, and aliphatic diisocyanates such as hexamethylene diisocyanate and 2,2'-trimethylhexamethylene diisocyanate. Examples of the polymerizable monomer having hydroxyl group(s) are (meth)acrylates having a hydroxy group such as β-hydroxyethyl (meth)acrylate, β-hydroxypropyl (meth)acrylate, β-hydroxylauryl (meth)acrylate, an adduct of ε-caprolactone and β-hydroxyethyl (meth)acrylate.

Although the reaction between the compound having an —NCO group and the compound having an —OH group proceeds in the absence of any catalyst, a conventional catalyst for the reaction of —NCO with —OH groups, for instance, a tertiary amine such as triethyl amine, organic metal compounds such as dibutyltin dilaurate and dibutyltin diacetate or tin chlorides may be used.

It is preferable to use the polyurethane (meth)acrylate (A) in an amount of from 20 to 70%, preferably from 30 to 60%, by weight of the amount of the resin composition.

The amount of the diester of (meth)acrylic acid (B) contained in the resin composition according to the present invention is preferably from 5 to 50%, particularly from 20 to 40%, by weight of the resin composition.

Although various monoethylenically unsaturated monomers (C) can be used in the resin composition according to the present invention, it is preferable to use a monomer which has a homopolymer having a glass-transition temperature as high as possible, for example, di-cyclopentadieneoxyethyl acrylate FA—512A, (made by HITACHI KASEI Co., Ltd.), dicyclopentadiene acrylate (FA—511A, made by HITACHI KASEI Co., Ltd.), hydrogenated-dicyclopentadiene acrylate (FA—513A, made by HITACHI KASEI Co., Ltd.), isobornyl (meth)acrylate, (meth)acrylate of hydrogenated β-naphthol, (meth)acrylate of tricyclodecanemethylol, phenyloxyethyl (meth)acrylate, tetrahydrofurfuryl (meth)acrylate, adamanthane (meth)acrylate and N-vinylpyrrolidone.

Of the monoethylenically unsaturated monomers, hydrogenated-dicyclopentadiene acrylate (FA—513A, made by HITACHI KASEI Co., Ltd.) and dicyclopentadieneoxyethyl acrylate (FA—512A, made by HITACHI KASEI Co., Ltd.) are particularly preferable. The amount of the monoethylenically unsaturated monomer used according to the present invention is preferably from 10 to 50%, more preferably from 20 to 40%, by weight of the resin composition.

The initiator of photopolymerization (D) optionally contained in the resin composition according to the present invention may be any known initiator of photopolymerization. However, it is required that the initiator has excellent stability after being compounded in the resin composition.

As such an initiator of photopolymerization, for instance, benzoin alkyl ethers such as benzoin ethyl ether, benzoin isobutyl ether and benzoin isopropyl ether, acetophenones such as 2,2-diethoxyacetophenone and 4'-phenoxy-2,2-dichloroacetophenone, propiophenones such as 2-hydroxy-2-methylpropiophenone, 4'-isopropyl-2-hydroxy-2-methylpropiophenone and 4'-dodecyl-2-hydroxy-2-methyl-propiophenone, benzyldimethylketal, 1-hydroxycyclohexyl phenyl ketone, anthraquinones such as

2-ethylanthraquinone and 2-chloroanthraquinone and thioxanthones may be mentioned. Of these initiators of photopolymerization (D), any one may be used singly or two or more of them may be used after mixing together in any ratio. The amount of the initiator used in the resin composition according to the present invention is usually not more than 10% by weight of the amount of the resin composition.

In the case where the resin composition according to the present invention is used for coating optical glass fibers, the initiator of photopolymerization (D) is an indispensable component of the resin composition, and in such a case the amount of the initiator of photopolymerization in the resin composition is preferably from 0.1 to 10%, more preferably 1 to 5%, by weight of the amount of the resin composition.

In addition, polymerizable monomer(s) such as polyfunctional acrylate compounds such as polyethylene glycol di(meth)acrylate, polypropylene glycol di(meth)acrylate and trimethylolpropane triacrylate, epoxyacrylate or polyester acrylate may be further added to the resin composition according to the present invention, if necessary. Further, resin(s) for modification and various adjuvant(s) may be added. As the resin for modification, epoxy resins, polyurethanes, polybutadienes, polyethers, polyamideimides, silicone resins and phenol resins may be mentioned. As the adjuvant, organic silicon compounds, surfactants and polymerization-inhibitors may be mentioned.

Although the resin composition according to the present invention may be used as a protective coating for plastic articles, it is particularly useful as a coating agent for optical glass fibers, especially when it is curable by ultraviolet rays; it is coated on the surface of the primary coating or the buffer coating on the surface of the optical glass fibers.

The present invention also provides optical glass fiber(s) coated with a resin composition as herein described.

In the case where the resin composition according to the present invention is used as a coating agent for optical glass fibers, the diesters of acrylic acid are preferable to the diesters of methacrylic acid as component (B) in the composition.

A suitable coating method of the optical glass fibers is the dies coating method. In such a case, after wire-drawing the base material of the optical glass, a primary coating agent is coated thereonto and, after curing the thus coated primary coating agent by irradiation of ultraviolet rays, the resin composition according to the present invention is coated as the top coating agent onto the primary preferably coating, at a thickness of from 20 to 300 µm.

The resin composition according to the present invention is easily cured by irradiation with ultraviolet rays, and such a curing may be carried out according to conventional methods. For instance, the thus coated resin composition is cured by irradiation of ultraviolet rays from a low-pressure mercury lamp, a high-pressure mercury lamp or a xenon lamp.

The present invention will be further explained in the following Examples, the part(s) in the Examples being part(s) by weight.

## EXAMPLES

### Synthesis of an adduct of tricyclodecanedimethylol and ε-caprolactone

#### Synthetic Example 1

In a 2 litre-reaction vessel equipped with a stirrer, a temperature controller, a thermometer and a reflux condenser, 942.2 parts of tricyclodecanedimethylol, 547.2 parts of ε-caprolactone and 0.27 part of isopropyl titanate were introduced, and the mixture was heated to a temperature of 150 to 160°C in a nitrogen atmosphere to carry out a reaction until the amount of unreacted ε-caprolactone was less than 1% by weight. The thus obtained adduct was a pale yellow liquid having a hydroxy value of 360.5 and an acid value of 1.2. As a result of molecular weight determination, it was shown that the thus obtained adduct of tricyclodecanedimethylol and ε-caprolactone had about one ε-caprolactone unit in average in one molecule of the adduct.

#### Synthetic Example 2

In the same reactor as in Synthetic Example 1, 588.8 parts of tricyclodecanedimethylol, 684.8 parts of ε-caprolactone and 0.34 part of stannous chloride were introduced, and the mixture was heated to a temperature of 110 to 120°C in a nitrogen atmosphere to carry out the reaction until the amount of unreacted ε-caprolactone was less than 1% by weight.

The thus obtained adduct was a pale yellow liquid having a hydroxy value of 266.5 and an acid value of 1.5. As a result of molecular weight determination, it was shown that the thus obtained adduct of tricyclodecanedimethylol and ε-caprolactone had about 2 ε-caprolactone units in one molecule of the adduct in average.

### Synthesis of a diester of (meth)acrylic acid

#### Example 1

In a 2-liter-reaction vessel equipped with a stirrer, a temperature controller, a thermometer, a reflux condenser and a separator, 470.3 parts of the 1:1 adduct of tricyclodecanedimethylol and ε-caprolactone obtained in Synthetic Example 1, 262 parts of acrylic acid, 7.86 parts of sulfuric acid, 1.98 parts of hydroquinone, 364 parts of benzene and 90.6 parts of cyclohexane were introduced, and the mixture was heated to a temperature of 81 to 89°C, thereby carrying out the reaction. The water formed was distilled together with the solvent, condensed and removed from the reaction system while the condensed solvent

was returned to the reaction vessel. When 54.6 parts of water were formed, the reaction mixture was cooled. After dissolving the reaction mixture in 809 parts of benzene and 202.4 parts of cyclohexane and neutralizing the solution with an aqueous 20% solution of sodium hydroxide, the neutralized solution was washed three times with each 500 parts of an aqueous 20% solution of sodium chloride. By distilling the solvent off from the thus washed solution under a reduced pressure, 523.4 parts of a pale yellow liquid having the following properties were obtained.

specific gravity (25°C): 1.105
viscosity (25°C): 0.2417 Pas (241.7 cP)
saponification value: 401.3 mgKOH/g
acid value: 0.02 mgKOH/g
refractive index (20°C): 1.4975

| Elementary analytical data: | C(%) | H(%) |
|---|---|---|
| | 68.89 | 8.20 |

The result of measurement of absorption spectrum of high resolution nuclear magnetic resonance (NMR) is shown below.

| No. of Peak | Absorption frequency (Hz) | No. of Peak | Absorption frequency (Hz) |
|---|---|---|---|
| 1 | 11739.6 | 25 | 2809.3 |
| 2 | 11246.0 | 26 | 2789.5 |
| 3 | 8831.1 | 27 | 2747.6 |
| 4 | 8703.3 | 28 | 2738.8 |
| 5 | 5263.8 | 29 | 2725.6 |
| 6 | 5233.0 | 30 | 2663.9 |
| 7 | 5200.0 | 31 | 2633.0 |
| 8 | 4662.3 | 32 | 2586.8 |
| 9 | 4651.3 | 33 | 2309.1 |
| 10 | 4622.7 | 34 | 2254.0 |
| 11 | 4594.0 | 35 | 2214.4 |
| 12 | 4587.4 | 36 | 2205.6 |
| 13 | 4349.5 | 37 | 2086.6 |
| 14 | 4334.0 | 38 | 2060.2 |
| 15 | 3366.7 | 39 | 1983.0 |
| 16 | 3318.3 | 40 | 1916.9 |
| 17 | 3089.1 | 41 | 1901.5 |
| 18 | 3040.6 | 42 | 1883.9 |
| 19 | 3016.4 | 43 | 1853.0 |
| 20 | 3001.0 | 44 | 1727.4 |
| 21 | 2967.9 | 45 | 1705.4 |
| 22 | 2912.9 | 46 | 1663.5 |
| 23 | 2895.2 | 47 | 1656.9 |
| 24 | 2826.9 | | |

In the NMR determination, tetramethylsilane was used as the standard substance, and chloroform was used as the solvent for the specimen. After carrying out the determination of $^1H$ signals and $^{13}C—H$ coupling signals $^{13}C—D$ coupling signals were determined to obtain identification results. Of the absorptions, Nos. 5, 6 and 7 show the absorption by the solvent.

Example 2

In the same reaction vessel as used in Example 1, 509.5 parts of the 1:2 adduct of tricyclodecanedimethylol and ε-caprolactone obtained in Synthetic Example 2, 207.4 parts of acrylic acid, 6.2 parts of sulfuric acid, 1.6 parts of hydroquinone, 384 parts of benzene and 96 parts of cyclohexane were introduced, and the reaction was carried out at a temperature of 80 to 87°C in the same manner as in Example 1 until 57.6 parts of water were formed. After dissolving the reaction mixture in 1065 parts of benzene and 266.3 parts of cyclohexane, and neutralizing the solution with an aqueous 20% solution of sodium hydroxide, the thus neutralized solution was washed three times with each 400 parts of an aqueous 20% solution of sodium chloride. By distilling the solvent off from the thus washed solution under a reduced pressure, 743.1 parts of a pale yellow liquid were obtained. The liquid had the following properties.

specific gravity (25°C): 1.102
viscosity (25°C): 0.4192 Pas (419.2 cP)
saponification value: 420.9 mgKOH/g
acid value: 0.04 mgKOH/g
refractive index (20°C): 1.4925
Elementary analytical data: C(%) 67.66, H(%) 8.32

The results of NMR measurement are as follows.

| No. of Peak | Absorption frequency (Hz) | No. of Peak | Absorption frequency (Hz) |
|---|---|---|---|
| 1 | 11732.9 | 22 | 2824.7 |
| 2 | 11239.4 | 23 | 2809.3 |
| 3 | 8828.9 | 24 | 2787.3 |
| 4 | 8701.1 | 25 | 2738.8 |
| 5 | 5263.8 | 26 | 2725.6 |
| 6 | 5233.0 | 27 | 2661.7 |
| 7 | 5200.0 | 28 | 2630.8 |
| 8 | 4662.3 | 29 | 2586.8 |
| 9 | 4620.5 | 30 | 2304.7 |
| 10 | 4607.2 | 31 | 2254.0 |
| 11 | 4585.2 | 32 | 2214.4 |
| 12 | 4349.5 | 33 | 2086.6 |
| 13 | 4334.0 | 34 | 2060.2 |
| 14 | 3357.9 | 35 | 1983.0 |
| 15 | 3316.1 | 36 | 1916.9 |
| 16 | 3089.1 | 37 | 1883.9 |
| 17 | 3038.4 | 38 | 1857.4 |
| 18 | 3016.4 | 39 | 1727.4 |
| 19 | 2967.9 | 40 | 1705.4 |
| 20 | 2912.9 | 41 | 1661.3 |
| 21 | 2895.2 | 42 | 0.0 |

Of the absorptions shown above, Nos. 5, 6 and 7 are the absorption peaks of the solvent.

Example 3

According to the same procedure as in the Synthetic Examples, an adduct of tricyclodecanedimethylol and ε-caprolactone having about four ε-caprolactone units in average in one molecule thereof was synthesized, and after introducing 502.8 parts of the thus synthesized adduct, 133.2 parts of acrylic acid, 4.0 parts of sulfuric acid, one part of phenothiazine, 384 parts of benzene and 96 parts of cyclohexane into the same reaction vessel as in Example 1, the reaction was carried out at a temperature of 80 to 88°C in the same manner as in Example 1 until 27.7 parts of water were formed.

After dissolving the reaction mixture in 720 parts of benzene and 180 parts of cyclohexane and neutralizing the solution with an aqueous 20% solution of sodium hydroxide, the thus neutralized solution was washed three times with each 400 parts of an aqueous 20% solution of sodium chloride. By distilling the solvent off from the thus washed solution under a reduced pressure, 510.9 parts of a pale yellow liquid were obtained. The thus obtained liquid had the following properties.

specific gravity (25°C): 1.0985
viscosity (25°C): 0.561.3 Pas (561.3 cP)
saponification value: 441.3 mgKOH/g
acid value: 0.02 mgKOH/g
refractive index (20°C): 1.4879

| | C(%) | H(%) |
|---|---|---|
| Elementary analytical data: | 66.31 | 8.49 |

The result of NMR measurement:

| No. of Peak | Absorption frequency (Hz) | No. of Peak | Absorption frequency (Hz) |
|---|---|---|---|
| 1 | 11735.1 | 24 | 2899.6 |
| 2 | 11241.6 | 25 | 2826.9 |
| 3 | 8831.1 | 26 | 2809.3 |
| 4 | 8703.3 | 27 | 2789.5 |
| 5 | 5268.3 | 28 | 2756.4 |
| 6 | 5235.2 | 29 | 2738.8 |
| 7 | 5202.2 | 30 | 2727.8 |
| 8 | 4684.4 | 31 | 2663.9 |
| 9 | 4651.3 | 32 | 2633.0 |
| 10 | 4622.7 | 33 | 2589.0 |
| 11 | 4609.4 | 34 | 2306.9 |
| 12 | 4587.4 | 35 | 2256.2 |
| 13 | 4349.5 | 36 | 2212.2 |
| 14 | 4336.2 | 37 | 2088.8 |
| 15 | 3360.1 | 38 | 2060.2 |
| 16 | 3349.1 | 39 | 1985.2 |
| 17 | 3316.1 | 40 | 1916.9 |
| 18 | 3089.1 | 41 | 1886.1 |
| 19 | 3040.6 | 42 | 1859.6 |
| 20 | 3016.4 | 43 | 1727.4 |
| 21 | 3001.0 | 44 | 1707.6 |
| 22 | 2970.1 | 45 | 1663.5 |
| 23 | 2915.1 | 46 | 0.0 |

Of the absorptions shown above, Nos. 5, 6 and 7 are the absorption peaks of the solvent.

Example 4

After synthesizing an adduct of tricyclodecanedimethylol and ε-caprolactone having about 10 ε-caprolactone units in average in one molecule of the adduct according to the same procedure as in the Synthetic Examples, 642.3 parts of the thus synthesized adduct, 72.6 parts of acrylic acid, 7.5 parts of p-toluenesulfonic acid, 0.6 parts of phenothiazine, 560 parts of benzene and 140 parts of cyclohexane were introduced into the same reaction vessel as in Example 1. The reaction was carried out at a temperature of 81 to 88°C in the same manner as in Example 1 until 17.2 parts of water were formed. After dissolving the reaction mixture in 800 parts of benzene and 200 parts of cyclohexane, neutralizing the solution with an aqueous 20% solution of sodium hydroxide, and washing the thus neutralized solution three times with each 400 parts of an aqueous 20% solution of sodium chloride, the solvent in the solution was distilled off from the solution under a reduced pressure, thereby obtaining 539.1 parts of a pale yellow semi-solid substance having the following properties.

acid value: 0.02 mgKOH/g
saponification value: 465.1 mgKOH/g
Elementary analytical data:

| C(%) | H(%) |
|---|---|
| 64.75 | 8.65 |

The result of NMR measurement:

| No. of Peak | Absorption frequency (Hz) | No. of Peak | Absorption frequency (Hz) |
|---|---|---|---|
| 1 | 11739.6 | 22 | 2899.6 |
| 2 | 11243.8 | 23 | 2809.3 |
| 3 | 8833.3 | 24 | 2787.3 |
| 4 | 8701.1 | 25 | 2725.6 |
| 5 | 5261.6 | 26 | 2663.9 |
| 6 | 5230.8 | 27 | 2630.8 |
| 7 | 5197.7 | 28 | 2591.2 |
| 8 | 4684.4 | 29 | 2306.9 |
| 9 | 4651.3 | 30 | 2254.0 |
| 10 | 4585.2 | 31 | 2212.2 |
| 11 | 4351.7 | 32 | 2187.9 |
| 12 | 4336.2 | 33 | 2088.8 |
| 13 | 4261.3 | 34 | 2055.7 |
| 14 | 4221.7 | 35 | 1916.9 |
| 15 | 3346.9 | 36 | 1883.9 |
| 16 | 3313.9 | 37 | 1859.6 |
| 17 | 3086.9 | 38 | 1727.4 |
| 18 | 3038.4 | 39 | 1661.3 |
| 19 | 3016.4 | 40 | 1474.1 |
| 20 | 2967.9 | 41 | 0.0 |
| 21 | 2915.1 | | |

Of the absorptions shown above, Nos. 5, 6 and 7 are absorption peaks of the solvent.

Example 5

After synthesizing an adduct of tricyclodecanedimethylol and ε-caprolactone having about 4 ε-caprolactone units in average in one molecule of the adduct according to the same procedure as in the Synthetic Examples, 502.8 parts of the thus synthesized adduct, 159.1 parts of methacrylic acid, 4.0 parts of sulfuric acid, 0.5 part of hydroquinone, 0.5 part of phenothiazine and 470 parts of toluene were introduced into the same reaction vessel as in Example 1, and the reaction was carried out at a temperature of 108 to 115°C in the same manner as in Example 1 until 27.7 parts of water were formed.

After dissolving the reaction mixture in 1000 parts of toluene, neutralizing the solution with an aqueous 20% solution of sodium hydroxide and washing the thus neutralized solution three times with each 400 parts of an aqueous 20% solution of sodium chloride, the solvent in the solution was distilled off from the solution under a reduced pressure, thereby obtaining 545.6 parts of a pale yellow liquid having the following properties.

specific gravity (25°C): 1.090
viscosity (25°C): 0.6864 Pas (686.4 cP)
saponification value: 423.5 mgKOH/g
acid value: 0.01 mgKOH/g
refractive index (20°C): 1.4856

| Elementary analytical data: | C(%) | H(%) |
|---|---|---|
| | 66.97 | 8.71 |

The result of NMR measurement:

| No. of Peak | Absorption frequency (Hz) | No. of Peak | Absorption frequency (Hz) |
|---|---|---|---|
| 1 | 11735.1 | 26 | 3003.2 |
| 2 | 11323.1 | 27 | 2970.1 |
| 3 | 9236.5 | 28 | 2915.1 |
| 4 | 8853.1 | 29 | 2899.6 |
| 5 | 8831.1 | 30 | 2809.3 |
| 6 | 8703.3 | 31 | 2789.5 |
| 7 | 8472.0 | 32 | 2725.6 |
| 8 | 5268.3 | 33 | 2663.0 |
| 9 | 5235.2 | 34 | 2633.4 |
| 10 | 5204.4 | 35 | 2593.4 |
| 11 | 4684.4 | 36 | 2306.9 |
| 12 | 4668.9 | 37 | 2254.0 |
| 13 | 4651.3 | 38 | 2212.2 |
| 14 | 4640.3 | 39 | 2196.8 |
| 15 | 4609.4 | 40 | 2190.1 |
| 16 | 4585.2 | 41 | 2088.8 |
| 17 | 4360.5 | 42 | 2057.9 |
| 18 | 4336.2 | 43 | 1919.1 |
| 19 | 4219.5 | 44 | 1886.1 |
| 20 | 3349.1 | 45 | 1857.4 |
| 21 | 3329.3 | 46 | 1727.4 |
| 22 | 3316.1 | 47 | 1707.6 |
| 23 | 3089.1 | 48 | 1663.5 |
| 24 | 3040.6 | 49 | 1238.3 |
| 25 | 3018.6 | 50 | 0.0 |

Of the absorptions shown above, Nos. 8, 9 and 10 are absorption peaks of the solvent.

Application Example 1

The primary irritation index (P.I.I.), the rate of curing and the degree of water absorption of each of the diesters of (meth)acrylic acid produced by the method set forth above are shown in Table 1, and compared with those of 1,6-hexanediol diacrylate and neopentyl glycol diacrylate.

Methods for Measurements

The measurements were carried out as follows.

PII (primary irritation index)

PII of diesters of (meth)acrylic acid was obtained on the basis of the experiments described in Huntingdon Research Centre Report (Huntingdon Cambs., PE18 6ES, England).

Outlined Procedure

Six white strain rabbits were used as the test animals. Hair was removed with electric clippers from the dorsolumber region of each rabbit.

A 0.5 ml aliquot of the test substance was applied to the exposed skin site on each animal according to a patch test method, and the thus treated sites were occluded with an impermeable material such as rubber or cloth for approximately 24 hours.

After 24 hours of the application of the test substance, the occlusive dressing and patches were removed and the examination of the treated skin sites was made by assessing the dermal reactions on the basis of the numerical scoring system set forth below. The examination was also made after 72 hours of the application of the test substance. Further, the examination was made in the same manner as described above on the abraded skin site on each animal. The skin should be abraded to make minor incisions through the stratum corneum, not through the corium, or bleeding takes place.

The numerical scores for erythema and eschar formation and the numerical scores dor oedema formation, at both the 24 and 72 hours readings for both intact and abraded skin sites, were added together and divided by 4 to obtain the numerical score for each animal. The average score of 6 animals was calculated on the basis of 6 numerical scores for each animal. The thus obtained average score is referred to as Primary Irritation Index (PII).

Numerical Scoring System

| Erythema and eschar formation | Score |
|---|---|
| No erythema | 0 |
| Very slight erythema (barely perceptible) | 1 |
| Well-defined erythema | 2 |
| Moderate to severe erythema | 3 |
| Severe erythema (beet redness) to slight eschar formation (injuries in depth) | 4 |

| Odema formation | Score |
|---|---|
| No oedema | 0 |
| Very slight oedema (barely perceptible) | 1 |
| Slight oedema (edges of area well defined by definite raising) | 2 |
| Moderate oedema (raised approximately 1 mm) | 3 |
| Severe oedema (raised more than 1 mm and extending beyond the area of exposure) | 4 |

For the details of the above skin irritation test, refer to Draize, John. H., Woodard, Geoffrey and Calvery, Herbert O., "Methods for the Study of Irritation and Toxicity of Substances Applied Topically to the Skin and Mucous Membranes", J. Pharm. & Exp. Ther. 82, 337 (1944).

The Speed of Curing

Each of the diesters of (meth)acrylic acid obtained in Examples 1 to 5, 1,6-hexanediol diacrylate and neopentyl glycol diacrylate was dissolved in an epoxyacrylate resin obtained by esterifying EPIKOTE 828 (an epoxy resin of bisphenol A-type, made by Shell Petrochem. Co.) with acrylic acid, and to each of the thus prepared solutions, DAROCURE 1173 (made by Merk Co.) was added as a sensitizer in an amount of 3% by weight of the thus dissolved substance. The thus prepared composition was applied onto a sheet of polyvinyl chloride at a thickness of 25 µm with a roll-coater, and the thus coated sheet was irradiated by ultraviolet rays from a high-pressure mercury lamp (made by TOSHIBA Co., 2 kw) at a distance of 8 cm while passing the sheet under the mercury lamp. The speed of curing is expressed by the times required to lose the wet feeling by a finger.

The Degree of Water Absorption

To each of the novel diesters of (meth)acrylic acid obtained in Examples 1 to 5, 1,6-hexanediol diacrylate and neopentyl glycol diacrylate, 5% by weight of a sensitizer (IRUGACURE 184, made by CIBA-GEIGY Co.) was respectively added, and the thus prepared composition was poured into a quartz glass container 3.0 cm (length) × 3.0 cm (width) × 0.1 cm (depth). The container was irradiated by ultraviolet rays from a high-pressure mercury lamp and the thus cured composition was immersed in water at 20°C. After leaving for one week, the increment of the weight of the cured material was measured to calculate the degree of water-absorption of the specimen.

Table 1

| No. | Resin (% by weight) | Di(meth)acrylate (% by weight) | PII | Speed of curing | Degree of water-absorption (%) |
|---|---|---|---|---|---|
| 1 | epoxyacrylate (30) | 1,6-hexanediol diacrylate (70) | 6.2 | 22 | 0.80 |
| 2 | epoxyacrylate (30) | neopentyl glycol diacrylate (70) | 4.96 | 18.5 | 0.71 |
| 3 | epoxyacrylate (30) | diester obtained in Example 1 (70) | 1.5 | 6.7 | 0.50 |
| 4 | epoxyacrylate (30) | diester obtained in Example 2 (70) | 1.6 | 6.7 | 0.45 |
| 5 | epoxyacrylate (30) | diester obtained in Example 3 (70) | 1.2 | 6.7 | 0.28 |
| 6 | epoxyacrylate (30) | diester obtained in Example 4 (70) | — | 10.1 | 0.35 |
| 7 | epoxyacrylate (30) | diester obtained in Example 5 (70) | — | 13.4 | 0.42 |

[Production of Polyurethane (meth)acrylate (A)]

Production Example 1

Into a 2 l reactor equipped with a stirrer, a temperature controller, a thermometer and a reflux condenser, 253.1 parts of polypropylene glycol (molecular weight of about 2000 and a hydroxy value of 56.1), 251.3 parts of polyester polyol (PLACCEL L—220AL, a reaction product of neopentyl glycol, adipic acid and ε-caprolactone, made by DICEL Chem. Ind. Co., Ltd., molecular weight of about 2000 and the hydroxy value of 57.5) and 84.7 parts of isophorone diisocyanate were introduced, and the mixture was heated for 10 hours at a temperature of 80°C. The reaction mixture was cooled to 60°C, and 91.4 parts of ε-caprolactone-β-hydroxyethyl acrylate (PLACCEL FA—2, made by DAICEL Chem. Ind. Co., Ltd.), 0.3 part of methoquinone and 0.1 part of di-n-butyltin dilaurate were added to the reaction mixture, and the thus prepared mixture was heated to from 75 to 80°C to carry out the reaction until the reaction was completed, the end point being shown by the presence of less than about 0.1% of free isocyanate groups. The thus obtained product had the following physical constants:

viscosity (60°C): 0.1 Pas (110 poise)
refractive index (20°C): 1.4721

Production Example 2

In a similar reactor as in Production Example 1, 714 parts of polytetramethylene glycol (molecular weight of 2040 and a hydroxyl value of 55.0), 67.6 parts of neopentyl glycol and 444.6 parts of isophorone diisocyanate were introduced, and the content of the reactor was heated at a temperature of 80°C for 10 hours. Then the reaction mixture was cooled to 60°C and 239 parts of 2-hydroxyethyl acrylate; 0.7 part of methoquinone, and 0.3 parts of di-n-butyltin dilaurate were added to the reaction mixture. The mixture was heated at a temperature of 75 to 80°C until the reaction was completed, the end point of the reaction being

shown by the presence of less than about 0.1% free isocyanate groups. The reaction product showed the following physical constants:

viscosity (25°C): 0.244 Pas (244 poise)
refractive index (20°C): 1.4792

[Preparation of the resin composition]

Example A 1

By mixing 40 parts of polyurethane acrylate (A) obtained in Production Example 2, 25 parts of diester of acrylic acid (B) obtained in Example 2, 35 parts of dicyclopentadieneoxyethyl acrylate (FA—512A, made by HITACHI KASEI Co. Ltd.) (C), 5 parts of 1-hydroxycyclohexyl phenyl ketone (IRUGACURE 184, made by Ciba-Geigy Co.) (D) and 0.01 part of methylhydroquinone together, the resin composition A was prepared, the properties thereof and the properties of the cured material thereof being shown in Table 2.

Example A 2

The resin composition B was prepared by mixing 10 parts of polyurethane acrylate (A) obtained in Production Example 1, 40 parts of polyurethane acrylate (A) obtained in Production Example 2, 25 parts of the diester of acrylic acid (B) obtained in Example 1, 25 parts of hydrogenated dicyclopentadiene acrylate (FA—513A, made by HITACHI KASEI Co., Ltd.) (C), 3 parts of benzyl dimethyl ketal (IRUGACURE 651, made by Ciba-Geigy Co.) (D) and 0.01 part of methylhydroquinone together. The properties of the thus prepared resin composition and those of the cured material thereof are shown in Table 2.

Example A 3

The resin composition C was prepared by mixing 35 parts of polyurethane acrylate (A) obtained in Production Example 2, 40 parts of the diester of acrylic acid (B) obtained in Example 3, 10 parts of phenyloxyethyl acrylate (PO—A, made by KYOEI Oil and Fat Co. Ltd.) (C), 15 parts of dicyclopentadieneoxyethyl acrylate (FA—512A, made by HITACHI KASEI Co., Ltd.) (C), 3 parts of benzyl dimethyl ketal (D) and 0.01 part of methylhydroquinone together. The properties of the thus prepared resin composition C and those of the cured material thereof are shown in Table 2.

Example A 4

By mixing 10 parts of polyurethane acrylate (A) obtained in Production Example 1, 35 parts of polyurethane acrylate (A) obtained in Production Example 2, 25 parts of the diester of acrylic acid (B) obtained in Example 1, 20 parts of hydrogenated dicyclopentadiene acrylate (C), 10 parts of N-vinylpyrrolidone (C), 3 parts of benzyl dimethyl ketal (D) and 0.01 part of methylhydroquinone together, the resin composition D was prepared. The properties of the thus prepared resin composition D and those of the cured material thereof are shown in Table 2.

Example A 5

By mixing 40 parts of polyurethane acrylate (A) obtained in Production Example 2, 15 parts of the diester of acrylic acid (B) obtained in Example 2, 10 parts of the diester of acrylic acid (B) obtained in Example 3, 15 parts of hydrogenated dicyclopentadiene acrylate (C), 10 parts of N-vinylpyrrolidone (C), 10 parts of tetrahydrofurfuryl acrylate (C), 3 parts of benzyl dimethyl ketal (D) and 0.01 part of methylhydroquinone together, the resin composition E was prepared. The properties of the thus prepared resin composition E and those of the cured material thereof are shown in Table 2.

Comparative Example 1

As an ultraviolet ray-curable, top-coating composition for optical fiber to be compared to the resin composition prepared in Examples A 1 to A 5, DESOTO 950 × 042 commercialized by Desoto Chemical Co. was chosen. The properties of DESOTO 950 × 042 (referred to as the resin composition F) and those of the cured material thereof are also shown in Table 2 for comparison.

| | Resin Composition | | | | | |
|---|---|---|---|---|---|---|
| | A | B | C | D | E | F |
| Viscosity of the composition (25°C, cps) | 3300 | 4500 | 2200 | 1600 | 2300 | 18500 |
| Properties of Cured material | | | | | | |
| Hardness (Shore D) | 70 | 60 | 45 | 65 | 60 | 54 |
| Tensile strength (23°C, kg/mm$^2$) | 2.1 | 3.0 | 2.5 | 3.4 | 2.8 | 2.4 |
| Elongation at break (23°C, %) | 56 | 70 | 80 | 75 | 86 | 35 |
| Young's modulus (60°C, kg/mm$^2$) | 1.4 | 3.5 | 2.3 | 6.9 | 1.4 | 2.4 |
| Young's modulus (40°C, kg/mm$^2$) | 9.4 | 18.7 | 10.5 | 26.8 | 6.8 | 6.1 |
| Young's modulus (23°C, kg/mm$^2$) | 22.9 | 30.1 | 25.3 | 47.8 | 31.2 | 15.0 |
| Young's modulus (−20°C, kg/mm$^2$) | 97.9 | 72.1 | 69.1 | 95.7 | 95.2 | 77.1 |
| Degree of water-absorption (increment of weight, %) | 0.9 | 0.7 | 0.4 | 1.6 | 1.9 | 4.7 |

Determination of Shore Hardness D of the cured material

After curing each specimen of the resin compositions A, B, C, D, E and F by irradiating with ultraviolet rays from a 2 kw high-pressure mercury lamp at a distance of 8 cm under the lamp while moving the composition at a speed of 5 m/min, the specimen was obtained as a 250 µm thick sheet. The shore hardness D of the sheet was determined following the method of Japanese Industrial Standards (JIS) Z 2246.

Determination of Tensile strength (kg/mm$^2$), Elongation at break (%) and Young's modulus (kg/mm$^2$) of the specimen

Each sheet of the cured resin compositions A to F made by the same method as above was used for determination of the tensile strength, the elongation at break and the Young's modulus.

Determination of the degree of water-absorption

Each sheet of the cured resin compositions A to F made by the same method as above was immersed in pure water at 20°C for 24 hours. The difference between the weight after and before immersion of each specimen was measured and the degree of water-absorption was expressed in percentage of the increment of the weight due to the absorbed water.

Example A 6

The base material for optical glass fiber was heated to about 2000°C and spun into optical glass fibers of 125 µm outer diameter at a speed of 5 m/sec, and in the next successive step, a primary coating agent (a mixture of 50% by weight of polyurethane acrylate, 45% by weight of a monoacrylate of 1:1 adduct of tetrahydrofurfuryl alcohol and ε-caprolactone, and 5% by weight of an initiator of photopolymerization) was applied onto the thus spun optical glass fiber, and then the thus applied primary coating was cured by ultraviolet rays. Then, after applying each of the resin compositions A to B obtained in Examples A 1 to A 5 onto the thus primary-coated optical glass fiber, and thus top-coated optical glass fiber was irradiated by ultraviolet rays from a high-pressure mercury lamp.

Each of the thus obtained optical glass fiber did not show any change of transmission loss at a temperature as low as −60°C.

The diesters of (meth)acrylic acid according to the present invention have a low Primary Irritation Index, a relatively low viscosity and cure at a high speed.

The resin composition according to the present invention cures at a high speed and has an appropriate viscosity for application, and the resin coating obtained by curing the resin composition according to the present invention has large elongation and low water-absorption, and has an appropriate Young's modulus and hardness, namely it is suitable for top-coating of optical glass fibers for use in transmitting light.

**Claims**

1. A diester of (meth)acrylic acid represented by the formula:

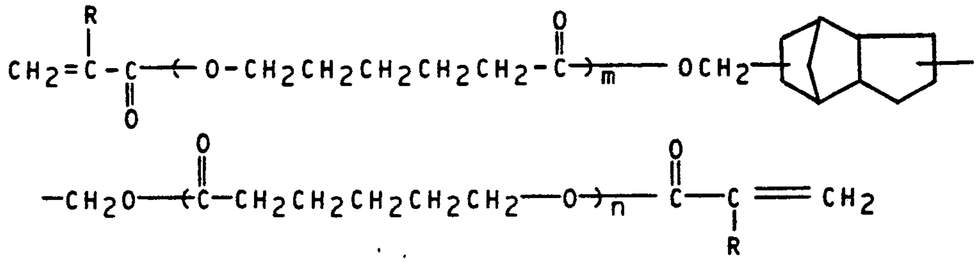

wherein the mean values of m and n are independently from 0 to 5, the mean value of m + n is from 1 to 10 and R represents a hydrogen atom or a methyl group.

2. A diester according to claim 1, wherein R represents a hydrogen atom.

3. A diester according to claim 1 or 2, wherein the mean values of m and n are independently from 0 to 3 and the mean value of m + n is from 1 to 6.

4. A resin composition comprising
(A) polyurethane(meth)acrylate(s);
(B) diester(s) of (meth)acrylic acid as claimed in any one of claims 1 to 3;
(C) monoethylenically unsaturated monomer(s); and
(D) initiator(s) of photopolymerization.

5. A composition according to claim 4, comprising from 20 to 70% by weight of polyurethane (meth)acrylate(s) (A); from 5 to 50% by weight of the diester(s) of (meth)acrylic acid (B); from 10 to 50% by weight of monoethylenically unsaturated monomer(s) (C); and from 0 to 10% by weight of initiator(s) of photopolymerization (D).

6. A composition according to claim 5, wherein the content of the said initiator(s) of photopolymerization (D) is from 0.1 to 10% by weight.

7. A composition according to any one of claims 4 to 6, wherein the average molecular weight of the or each polyurethane (meth)acrylate is from 500 to 5000.

8. Optical glass fiber(s) coated with a resin composition as claimed in any one of claims 4 to 7.

**Patentansprüche**

1. Diester der (Meth)acrylsäure, dargestellt durch die Formel

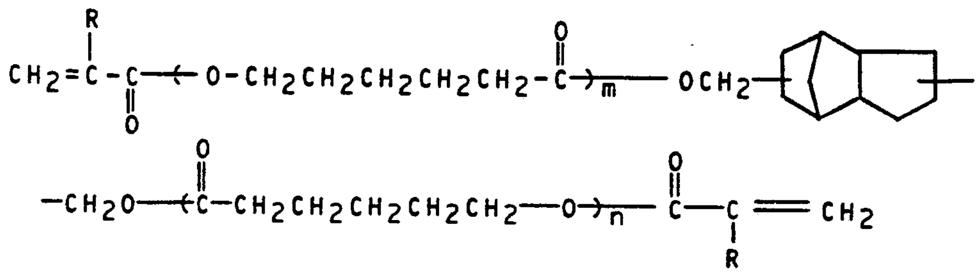

worin die Mittelwerte von m und n unabhängif voneinander 0 bis 5 betragen, der Mittelwert von m + n 1 bis 10 beträgt und R ein Wasserstoffatom oder eine Methylgruppe bedeutet.

2. Diester nach Anspruch 1, wobei R ein Wasserstoffatom bedeutet.

3. Diester nach Anspruch 1 oder 2, wobei die Mittelwerte von m und n unabhängig voneinander 0 bis 3 betragen und der Mittelwert von m + n 1 bis 6 beträgt.

4. Harzzusammensetzung, umfassend
(A) Polyurethan(meth)acrylat(e);
(B) Diester der (Meth)acrylsäure nach mindestens einem der Ansprüche 1 bis 3;
(C) monoethylenisch ungesättigtes Monomer(e); und
(D) Photopolymerisationsinitiator(en).

5. Zusammensetzung nach Anspruch 4, umfassend 20 bis 70 Gew.-% Polyurethan(meth)acrylat(e) (A); 5 bis 50 Gew.-% des (der) Diester der (Meth)acrylsäure (B); 10 bis 50 Gew.-% monoethylenisch ungesättigtes Monomer(e) (C); und 0 bis 10 Gew.-% Photopolymerisationsinitiator(en) (D).

6. Zusammensetzung nach Anspruch 5, wobei der Gehalt des (der) Photopolymerisationsinitiators(en) (D) 0,1 bis 10 Gew.-% beträgt.

7. Zusammensetzung nach mindestens einem der Ansprüche 4 bis 6, wobei das Durchschnittsmolekulargewicht des oder jedes Polyurethan(meth)acrylats 500 bis 5000 beträgt.

8. Optische Glasfaser(n), beschichtet mit einer Harzzusammensetzung nach mindestens einem der Ansprüche 4 bis 7.

**Revendications**

1. Diester de l'acide (méth)acrylique représenté par la formule:

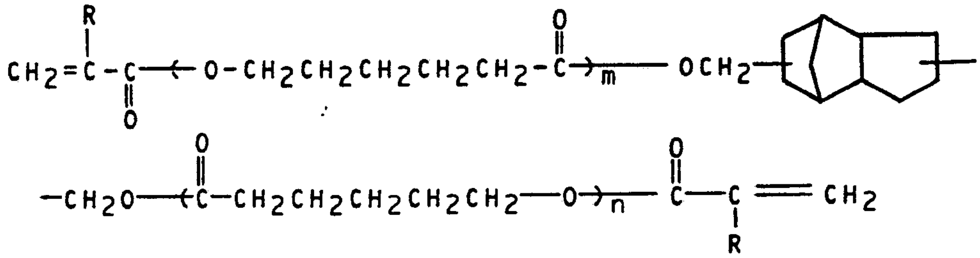

dans laquelle les valeurs moyennes de m et n sont indépendamment de 0 à 5, la valeur moyenne de m + n est de 1 à 10 et R représente un atome d'hydrogène ou un groupe méthyle.

2. Diester suivant la revendication 1, dans lequel R représente un atome d'hydrogène.

3. Diester suivant les revendications 1 ou 2, dans lequel les valeurs moyennes de m et n sont indépendamment de 0 à 3 et la valeur moyenne de m + n est de 1 à 6.

4. Composition de résine comprenant:

(A) un ou plusieurs polyuréthanes (méth)acryliques;

(B) un ou plusieurs diesters de l'acide (méth)acrylique tels que revendiqués dans l'une quelconque des revendications 1 à 3;

(C) un ou plusieurs monomères à insaturation monoéthylénique; et

(D) un ou plusieurs agents d'amorçage de la photopolymérisation.

5. Composition suivant la revendication 4, comprenant de 20 à 70% en poids d'un ou plusieurs polyuréthanes (méth)acryliques (A); de 5 à 50% en poids d'un ou plusieurs diesters de l'acide (méth)acrylique (B); de 10 à 50% en poids d'un ou plusieurs monomères à insaturation monoéthylénique (C); et de 0 à 10% en poids d'un ou plusieurs agents d'amorçage de la photopolymérisation (D).

6. Composition suivant la revendication 5, dans laquelle la teneur de ce ou ces agents d'amorçage de la photopolymérisation (D) est de 0,1 à 10% en poids.

7. Composition suivant l'une quelconque des revendications 4 à 6, dans laquelle la masse moléculaire moyenne du ou de chaque polyuréthane (méth)acrylique est de 500 à 5 000.

8. Fibre(s) de verre optique(s) revêtue(s) d'une composition de résine suivant l'une quelconque des revendications 4 à 7.